# EUROPEAN PATENT SPECIFICATION

(11) **EP 3 517 096 B1**
(45) Date of publication and mention of the grant of the patent: **04.11.2026**
(21) Application number: 17853321.2
(22) Date of filing: 01.09.2017
(51) Int. Cl.: A61K 8/37, A61K 8/92, A61K 8/36, A61K 8/66, A61K 8/06, A61Q 19/00, A23D 9/04, C11C 3/10

(54) **SKIN TEMPERATURE-SENSITIVE FAT COMPOSITION AND A COSMETIC COMPOSITION**
HAUTTEMPERATUREMPFINDLICHE FETTZUSAMMENSETZUNG UND KOSMETISCHE ZUSAMMENSETZUNG
COMPOSITION DE GRAS SENSIBLE À LA TEMPÉRATURE DE LA PEAU ET COMPOSITION COSMÉTIQUE

(30) Priority: 23.09.2016 KR 20160122415
(43) Date of publication of application: 31.07.2019
(73) Proprietor: CJ Cheiljedang Corporation, Seoul 04560 (KR)
(72) Inventor: KIM, Mijung, Suwon-si Gyeonggi-do 16593 (KR); LEE, Yunjeong, Seongnam-si Gyeonggi-do 13595 (KR); PARK, Seung Won, Yongin-si Gyeonggi-do 16894 (KR); OH, Han Na, Suwon-si Gyeonggi-do 16509 (KR); LEE, Sang Bum, Seoul 08202 (KR); CHO, Seong Jun, Seoul 04971 (KR)
(74) Representative: Regimbeau
(86) International application number: PCT/KR2017/009623
(87) International publication number: WO 2018/056607

(56) References cited:
- EP-A1- 2 561 763
- EP-A2- 2 583 561
- WO-A2-2011/159024
- FR-A1- 2 772 391
- KR-A- 20050 100 500
- KR-B1- 101 229 383
- KR-B1- 101 344 491
- US-A- 4 276 322
- LIU KUAN-JU ET AL: "Synthesis of cocoa butter equivalent by lipase-catalyzed interesterification in supercritical carbon dioxide", JOURNAL OF THE AMERICAN OIL CHEMISTS' SOCIETY (JAOCS), SPRINGER, DE, vol. 74, no. 11, 1 November 1997 (1997-11-01), pages 1477 - 1482, XP002246317, ISSN: 0003-021X, DOI: 10.1007/S11746-997-0257-Z
- CHONG C N ET AL: "FRACTIONATION PROCEDURES FOR OBTAINING COCOA BUTTER-LIKE FAT FROM ENZYMATICALLY INTERESTERIFIED PALM OLEIN", JOURNAL OF THE AMERICAN OIL CHEMISTS' SOCIETY (JAOCS), SPRINGER, DE, vol. 69, no. 2, 1 February 1992 (1992-02-01), pages 137 - 140, XP000261553, ISSN: 0003-021X, DOI: 10.1007/BF02540563

## Description

### [Technical Field]

The present disclosure relates to a lipid composition comprising at least 40 wt% of saturated acid-unsaturated acid-saturated acid (SUS) based on the total weight of the lipid composition, wherein the SUS comprises 58 parts by weight to 74 parts by weight of 1-palmitoyl-2-oleoyl-3-stearoyl glycerol (POS) based on 100 parts by weight of SUS, wherein the lipid composition further comprises a vegetable lipid which comprises 0 wt% to 5 wt% of solid fat content (SFC) at 30°C, and wherein a ratio of solid fat content (SFC) at a temperature of 20°C to 25°C compared to that of SFC at 35°C is 10 to 35, a cosmetic composition comprising the same, and a method for preparing the lipid composition.

### [Background Art]

Symmetric lipids, which are a type of saturated acid-unsaturated acid-saturated acid (SUS), are mostly contained in naturally existing cocoa butter, palm mid fraction (PMF), Illipe butter, shea stearin, *etc.,* and a major symmetric lipid is triglycerides consisting of three ingredients, which are 1,3-palmitoyl-2-oleoyl glycerol (POP), POS, and 1,3-distearoyl-2-oleoyl glycerol (SOS). However, the composition of the triglycerides mostly has predominant POP and/or SOS contents compared with POS content, except palm mid fraction (POP 57%, POS 11%, SOS 2%); Illipe butter (POP 9%, POS 29%, SOS 42%); shea stearin (POP 3%, POS 10%, SOS 63%); and cocoa butter (POP 16%, POS 38%, SOS 23%) (Confectionery Fats Handbook, ISBN 0-9531949-4-9, p.230).

Symmetric lipids exist in various crystalline forms according to environmental factors, although they contain triglyceride in the same composition, which is defined as polymorphism. Each crystalline form may have different physical properties. POPs have a melting point in a range of 15°C to 37°C depending on the crystalline form. The melting point of the β₂-3 type, which is a major crystalline form of POP, is 35°C. POSs have a melting point in a range of 19°C to 36°C. The melting point of the β-3 type, which is a major crystalline form of POS, is 35.5°C. SOSs have a melting point in a range of 23.5°C to 43°C. The melting point of the β₂-3 type, which is a major crystalline form of SOS, is 41°C. (Confectionery Fats Handbook, ISBN 0-9531949-4-9, p.54). Accordingly, the three types of the symmetric lipids have different physical properties from one another. For example, POP is relatively soft, whereas SOS is relatively hard. As POS has properties between those of POP and SOS, it has superior utility among the symmetric lipids.

As previously mentioned, however, naturally existing raw materials commonly have low POS contents. Although the POS content in cocoa butter is 38%, natural materials have several disadvantages. For example, there has been a steady rise in the sustainability-related issue of the supply of POS from nature without destruction of the environment. Moreover, there may be differences in quality depending on environmental factors, and due to severe price fluctuations depending on crop conditions and especially high cost, the unit cost of production is increasing. To resolve such disadvantages, POS can be prepared via modification of the lipids using a chemical or enzymatic method; however, the chemical method may cause environmental pollution due to use of chemical catalysts, and has difficulty in preparing POS in a high content due to lack of positional specificity during reaction. In contrast, the preparation of POS by the enzymatic method has no risk of environmental pollution, and is advantageous in that a high content of POS can be prepared because the enzymes have positional specificity. Further, the enzymatic method can remedy the disadvantages of the natural materials, such as unstable quality and high prices, and thereby consistently maintain and manage quality, and further is economically competitive due to a low cost. European Patent Applications EP-A-2 561 763 and EP-A-2 583 561 disclose fat compositions obtained from enzymatic trans-esterification and having a high content of 1-palmitoyl-2-oleoyl-3-stearoyl glycerol (POS).

The applicant has conducted studies on symmetric lipid compositions, and as a result, has completed preparation of a lipid composition which provides an excellent feel upon application to skin and excellent tactility by exhibiting a drastic reduction of a solid fat content at a temperature in a range of 30°C to 35°C, which is similar to the temperature of human skin.

### [Disclosure]

### [Technical Problem]

An objective of the present disclosure is to provide a lipid composition as required in claim 1 comprising at least 40 wt% of saturated acid-unsaturated acid-saturated acid (SUS) based on the total weight of the lipid composition, wherein the SUS comprises 58 parts by weight to 74 parts by weight of 1-palmitoyl-2-oleoyl-3-stearoyl glycerol (POS) based on 100 parts by weight of SUS.

Another objective of the present disclosure is to provide a cosmetic composition comprising the lipid composition.

A further objective of the present disclosure is to provide a method for preparing the lipid composition comprising performing transesterification by mixing a fatty acid or a derivative thereof; a vegetable lipid; and lipase.

### [Technical Solution]

The applicant has conducted studies on symmetric lipid compositions, and as a result, has completed preparation of a lipid composition which provides an excellent feel upon application to skin and excellent tactility by exhibiting a drastic reduction of a solid fat content at a temperature in a range of 30°C to 35°C, which is similar to the temperature of human skin.

### [Advantageous Effects]

The lipid composition of the present disclosure is dissolved at a temperature similar to a skin temperature, and thereby its cosmetic formulation can provide excellent feel and tactility with less stickiness upon its application to the skin. Further, the lipid composition of the present disclosure has remarkably high shelf stability, and accordingly can maintain the quality even when stored for a long period of time.

### [Best Mode for Carrying Out the Invention]

To achieve the objectives, an exemplary embodiment provides a lipid composition comprising at least 40 wt% of saturated acid-unsaturated acid-saturated acid (SUS) based on the total weight of the lipid composition, wherein the SUS comprises 58 parts by weight to 74 parts by weight of 1-palmitoyl-2-oleoyl-3-stearoyl glycerol (POS) based on 100 parts by weight of SUS, wherein the lipid composition further comprises a vegetable lipid which comprises 0 wt% to 5 wt% of solid fat content (SFC) at 30°C, andwherein a ratio of solid fat content (SFC) at a temperature of 20°C to 25°C compared to that of SFC at 35°C is 10 to 35.

An embodiment of the present disclosure provides a cosmetic composition comprising the lipid composition.

An embodiment of the present disclosure provides a method for preparing the lipid composition comprising performing transesterification by mixing a fatty acid or a derivative thereof; a vegetable lipid; and lipase.

Hereinafter, the present disclosure will be described in detail. The lipid composition has a technical feature in variance of its solid fat content depending on the temperature and its high oxidation stability. The lipid composition exhibits a drastic reduction of the solid fat content at a temperature in a range of 30°C to 35°C, which is similar to that of the human skin, and accordingly may immediately be dissolved and absorbed into the skin upon its application to the skin, which results in an excellent feel and its use in cosmetic compositions. Further, the lipid composition can be prepared in an eco-friendly method by reacting a vegetable lipid and a fatty acid or a derivative thereof in the presence of an enzymatic catalyst.

The lipid composition comprises at least 40 wt% of saturated acid-unsaturated acid-saturated acid (SUS) based on the total weight thereof. Specifically, the content of SUS may be 40 wt% to 100 wt%, 50 wt% to 95 wt%, 60 wt% to 90 wt%, 65 wt% to 85 wt%, or 70 wt% to 80 wt%. The lipid composition comprises 58 parts by weight to 74 parts by weight of POS based on 100 parts by weight of SUS Additionally, the lipid composition may contain at least 60 wt% of a saturated fatty acid, such as palmitic acid and stearic acid, and at least 33 wt% of unsaturated fatty acid, such as oleic acid and linoleic acid, but is not limited thereto. The lipid composition comprises a high content of SUS, particularly POS among SUS, and thereby has a feature that the content of solid fat of the composition is high at room temperature and low at 35°C.

According to an exemplary embodiment, a variation of red color of the lipid composition, measured in accordance with ISO 15305 (AOCS Cc13b-45), may be 0.1 R or less at 25°C for five weeks. Stability of the lipid composition can be evaluated by measuring a color value of the lipids and analyzing a change or variation of the value. For example, the color value of the lipid composition can be measured in accordance with ISO 15305 (AOCS Cc13b-45), and the measured value can be analyzed with respect to R (Red) or Y (Yellow) value using the AOCS or Lovibond method. Methods for measuring and analyzing the color value are not limited to the previously described methods, and methods or machinery widely known in the art can be used without limitation. For example, a variation of red color of the lipid composition, measured in accordance with ISO 15305 (AOCS Cc13b-45), may be 0.1 R or less at 25°C for five weeks. The lipid composition maintains its color and formulation, and quality of the lipids during its long-term storage at room temperature, and thus is very suitable for use as a lipid for cosmetics or foods.

According to an exemplary embodiment, a variation of red color of the lipid composition, measured in accordance with ISO 15305 (AOCS Cc13b-45), may be 0.3 R or less or 0.2 R or less at 80°C for six hours. The lipid composition rarely shows a color change even when it is exposed to the air at a high temperature, compared with a natural lipid such as shea butter, and thus can overcome the disadvantage that conventional or natural lipids have in relation to oxidation stability.

According to an exemplary embodiment, the lipid composition has a solid fat content (SFC) of 45 wt% to 75 wt% at 20°C, and of 0.5 wt% to 3 wt% at 35°C. The SFC may be a value measured in accordance with ISO 8292-2.

An SFC ratio of the lipid composition at a temperature of 20°C to 25°C is higher than that at 35°C. For example, in the case of shea butter, which is a representative butter conventionally used for cosmetics, ratios of the SFC at 25°C or 20°C to the SFC at 35°C are 2 to 4, whereas in the case of the lipid composition, a ratio of the SFC at 20°C to 25°C to the SFC at 35°C is 10 to 35. More specifically, a ratio of the SFC of the lipid composition at 20°C to the SFC at 35°C may be 10 to 35, 14 to 32, 16 to 30, 18 to 28, or 20 to 26. Further, a ratio of SFC of the lipid composition at 25°C to the SFC at 35°C is 10 to 35, 12 to 30, 14 to 28, 16 to 26, or 18 to 24.

Meanwhile, the lipid composition has a low SFC of 0.5 wt% to 10 wt% at 35°C, and a ratio of the SFC at 30°C to the SFC at 35°C may be 8 to 20 or 10 to 16. In contrast, shea butter has a high SFC of about 11.3 wt% at 35°C, and a ratio of the SFC at 30°C to the SFC at 35°C is as low as 2.93. In other words, the lipid composition stably maintains its solid phase at room temperature of 20°C to 25°C, and drastically changes its phase into liquid at a temperature of 30°C to 35°C, which is similar to the body temperature. In contrast, shea butter is in a half-solid phase at room temperature, and is not easily dissolved at the body temperature. Accordingly, when applied to the skin, the lipid composition rapidly changes its phase, and thus can provide ease in its application to the skin and/or an excellent feel, and can maintain its stable shape as a solid phase at room temperature.

According to an exemplary embodiment, the lipid composition may further comprise a vegetable lipid which has an SFC of 0 wt% to 5 wt% at 30°C. Unlimited examples of the vegetable lipid which can be added to the lipid composition may include palm oil, palm olein, palm stearin, shea olein, and shea stearin. The palm oil may be a natural material itself or a fraction thereof. One type of the vegetable lipid may be solely added, or a combination of two or more types of the vegetable lipids may be added. The vegetable lipid to the lipid composition may be added at the ratio of 1:1, specifically, up to 60:40, but is not limited thereto as long as the total mixture maintains the SUS content and POS/SUS ratio within the previously described range.

According to an exemplary embodiment, the present disclosure provides a cosmetic composition comprising the lipid composition.

As previously described, the lipid composition exhibits a remarkably low SFC at 35°C, and drastically reduces the SFC particularly at a temperature in the range of 30°C to 35°C, which is similar to the skin temperature. Accordingly, when the lipid composition is administered onto the skin, it rapidly changes its phase in response to the body temperature, and thus can provide ease in its application to the skin, excellent skin adhesion, moisture, *etc.,* and can be used as a cosmetic composition. Additionally, the lipid composition shows equivalent or superior properties compared to expensive shea butter, which is conventionally used for cosmetic compositions. Moreover, the lipid composition has excellent stability, thereby maintaining the quality without discoloration or precipitation even when stored for a long period of time, and thus is suitable for use in a cosmetic composition. The cosmetic composition may comprise 2 wt% to 80 wt% of the lipid composition. The content of the lipid composition may vary depending on types and ingredient composition of cosmetics to which the lipid composition is applied. For example, the content may be 2 wt% to 80 wt%, 5 wt% to 50 wt%, 7 wt% to 45 wt%, 10 wt% to 40 wt%, 15 wt% to 35 wt%, or 20 wt% to 30 wt%, but is not limited thereto.

The lipid composition contains at least 70 wt% of triglyceride which is a saturated acid-unsaturated acid-saturated acid (SUS), whereas shea butter contains about 45 wt% thereof. Additionally, in comparison of fatty acids of which the lipid composition and the shea butter are composed, the lipid composition contains at least 60 wt% of saturated fatty acid, such as palmitic acid and stearic acid, and at least 33 wt% of unsaturated fatty acid, such as oleic acid and linoleic acid. In contrast, shea butter contains at least 48 wt% of saturated fatty acid, such as palmitic acid and stearic acid, and at least 50 wt% of unsaturated fatty acid, such as oleic acid and linoleic acid. In other words, shea butter has a low content of triglyceride, and a high content of unsaturated fatty acid, resulting in a low SFC and poor oxidation stability at a temperature in the range of 20°C to 25°C, which is close to room temperature. In contrast, the lipid composition is characterized by having high oxidation stability as well as a high SFC even at room temperature.

The cosmetic composition may be prepared in a formulation selected from the group consisting of a solution, skin ointment, cream, foam, nourishing lotion, softening lotion, mask, softening liquid, milky liquid, makeup base, essence, lip balm, soap, liquid cleanser, bath preparation, sunscreen cream, sun oil, suspension, emulsion, paste, gel, lotion, powder, surfactant-containing cleanser, oil, powder foundation, emulsion foundation, wax foundation, patch, and spray, but is not limited thereto. The cosmetic composition of the present disclosure can provide excellent feel upon its application to skin, and thus is preferable for formulation in a product which is directly applied to the skin, such as skin ointment, cream, milky liquid, makeup base, soap, sunscreen cream, paste, gel, lotion, emulsion foundation, wax foundation, lip balm, but is not limited thereto.

Additionally, the cosmetic composition may further comprise at least one type of a cosmetically acceptable carrier mixed into general skin cosmetics. For example, fat content, water, surfactant, moisturizer, low grade alcohol, thickener, chelating agent, colorant, preservative, perfume, *etc.* may be appropriately mixed as a conventional ingredient, but the carrier is not limited thereto. A cosmetically acceptable carrier included in the cosmetic composition may be variously selected depending on its formulation. For example, when the formulation is an ointment, paste, cream, or gel, animal oil, vegetable oil, wax, paraffin, starch, tragacanth, a cellulose derivative, polyethylene glycol, silicone, bentonite, silica, talc, oxidized zinc, or a mixture thereof can be used as a carrier, but it is not limited thereto.

Or the cosmetic composition may be formulated as a functional cosmetic by further including an ingredient having effects on anti-oxidation, wrinkle improvement, anti-aging, and/or UV blocking. Active ingredients known in the art, which are used in functional cosmetics, can be used as the ingredient having effects on anti-oxidation, wrinkle improvement, anti-aging, and/or UV blocking without limitation.

Further, the lipid composition may be prepared through a process including transesterification by mixing a fatty acid or a derivative thereof, a vegetable lipid, and a lipase. The SUS content and ratio of POS to SUS of the lipid composition may be adjusted by controlling the ratio and/or reaction time of the used reactants.

For example, the fatty acid may be palmitic acid, stearic acid, arachidonic acid, or behenic acid. Meanwhile, the fatty acid derivative may be an alkyl ester derivative of a fatty acid, specifically, palmitic acid ethyl ester, stearic acid ethyl ester, arachidonic acid ethyl ester, behenic acid ethyl ester, palmitic acid methyl ester, stearic acid methyl ester, arachidonic acid methyl ester, or behenic acid methyl ester. The fatty acid or derivative thereof may be specifically stearic acid, stearic acid ethyl ester, stearic acid methyl ester, or a mixture thereof, but is not limited thereto.

In the preparation method of the present disclosure, a vegetable lipid itself or a fraction thereof can be used as the vegetable lipid. Unlimited examples of the vegetable lipid which can be used in the method for preparing the lipid composition of the present disclosure may include coconut oil, palm kernel oil, palm oil, canola oil, sunflower oil, soy bean oil, cotton seed oil, rice oil, corn oil, olive oil, shea fat, mango kernel fat, Borneo tallow(Shorea stenoptera or Pentadesma butyracea), sal(Shorea robusta), or kokum(Garcinia indica), but are not limited thereto, and may include all vegetable lipids used in the art. Specifically, it may be palm kernel oil, palm oil, or sunflower oil, but is not limited thereto.

Alternatively, by fractionating the vegetable lipid, a lipid fraction containing 1,3-dipalmitoyl-2-oleolglycerol (POP) may be used. The fractionation may be conducted through dry fractionation or solvent fractionation, but is not limited thereto, and a method for providing a POP-containing lipid having difference in contents of saturated and unsaturated fatty acids from raw materials of vegetable lipids known in the art may be used without limitation. For example, the fractionation method may selectively be used depending on characteristics of the raw materials of the vegetable lipids. For solvent fractionation, an organic solvent capable of dissolving raw lipids, such as hexane, acetone, methyl ethyl ketone, and ethanol, may be used without limitation. The vegetable lipid which can be used in the present disclosure may be a lipid containing POP of 35 wt% to 70 wt% and having an iodine value of 35 to 55.

For example, transesterification using the lipase is designed to prepare a symmetric triglyceride including a saturated fatty acid at the sn-1,3 positions and unsaturated fatty acid at the sn-2 position, and may be conducted by reacting an enzyme having specificity to the sn-1,3 positions at 30°C to 60°C for 1 to 30 hours, but is not limited thereto.

Unlimited examples of the enzyme having specificity to the sn-1,3 positions may include an enzyme isolated from *Rhizopus delemar, RhizoMucor miehei, Aspergillus miger, Rhizopus arrhizus, Rhizopus niveus, Mucor javanicus, Rhizopus javenicus, Rhizopus oxyzae, Thermomyces lanuginosus, etc.,* specifically, an enzyme derived from *Mucor miehei* or *Thermomyces lanuginosus,* but the enzyme is not limited thereto, and any enzyme used in the art which is specific to the sn-1,3 positions may be used. More specifically, a lipase derived from *Mucor miehei* can be used, but the lipase is not limited thereto.

In addition, after the transesterification, addition of a vegetable lipid which comprises 0 wt% to 5 wt% of SFC at 30°C may further be conducted, but the lipid is not limited thereto. Examples of the vegetable lipid which comprises 0 wt% to 5 wt% of SFC at 30°C are the same as previously described.

The lipid composition prepared by the above method has excellent miscibility with other cosmetic raw materials, and is suitable for use as a cosmetic raw material because of its equivalent or superior shelf stability, compared to expensive butter, e.g., shea butter, which is used in existing cosmetic compositions. Further, because of the high SUS and POS contents, the lipid composition is characterized by rapid melting at a temperature in the range of 30°C to 35°C, which is close to the skin temperature. Accordingly, the composition is rapidly dissolved and absorbed into the skin upon its application to skin, and thus is advantageous in that the composition can provide excellent feel and tactility with less stickiness.

### [Mode for Carrying Out the Invention]

Hereinafter, the present disclosure will be described in detail with accompanying exemplary embodiments. However, the exemplary embodiments disclosed herein are only for illustrative purposes and should not be construed as limiting the scope of the present disclosure.

### Example 1: Preparation of POS by enzymatic transesterification

### 1.1. Preparation of palm fraction

A palm fraction for use as a raw lipid was obtained through solvent fractionation. Specifically, 1 kg of palm oil was completely dissolved at 60°C, was mixed with 10 kg of acetone and sealed. The mixture was stirred so that the lipid is completely dissolved in acetone. The mixed solution was stirred at 30 rpm and 0°C for three hours, and then was crystallized. Vacuum filtration was then used to separate the mixed solution into palm stearin in a solid phase and palm olein in a liquid phase. A yield of palm olein was at least 60%, and an iodine value thereof was 60 or less.

The palm stearin obtained by the fractionation without acetone removed was completely dissolved at 40°C, and was mixed with additional acetone. The mixture was stirred at 30 rpm and 30°C and crystallized, followed by vacuum filtration, to be separated into crystallized fractions and palm mid fractions (PMFs). A yield of palm mid fractions was at least 30%, a POP (palmitic-oleic-palmotic) content was 35% to 70%, and an iodine value thereof was 35 to 55.

### 1.2. Preparation of POS through enzymatic reaction

Stearic acid and derivatives thereof were mixed with PMFs (POP 55%, iodine value 40) prepared according to Example 1.1 above at molar ratios of 1:1, 1:1.5, 1:2, 1:3, 1:4, and 1:5, to give the total weight of 2 kg. The mixture was subjected to transesterification with NOVOZYME 40086 (immobilized sn-1,3-specific lipase from *Mucor miehei*), which is an enzyme which accounts 5 wt% of the raw lipid and has specificity to the sn-1,3 positions, for 3 hours to 12 hours to prepare a lipid composition.

### Example 2: Isolation of lipid composition containing POS

The lipid composition prepared by the enzymatic transesterification according to Example 1 above was distilled to be isolated from a fatty acid ethyl ester. The fatty acid ethyl ester was removed using a four-column distillation tower under the conditions of vacuum pressure 2 mbar, bottom column temperature 235°C, and reflux ratio 1 to obtain a lipid composition having a high POS content.

### Example 3: Analysis of triglyceride composition of lipid composition

### 3.1. HPLC-ELSD analysis

Analysis of triglyceride of the lipid composition in regard to its type and content before/after the enzymatic transesterification were performed using High Performance Liquid Chromatography (HPLC). The specific conditions for the triglyceride analysis using HPLC are shown in Table 1 below. The structure of triglyceride in the lipid according to reaction conditions was analyzed using the HPLC-ELSD (Evaporative Light Scattering Detector) system. 30 µL of a sample and 10 mL of a solvent, which is a mixture of acetonitrile and dichloromethane at a ratio of 70:30, were filtered using a PEFE syringe filter (25 mm, 0.45 µL), and then was put into a 2 mL vial. 20 µL of the sample was then injected using an autosampler. Acetonitrile (Solvent A) and dichloromethane (Solvent B) were used, and a flow rate was 0.72 mL/min. Solvent gradient elution (A:B, v:v) was conducted at 70:30 for 25 minutes, and then a gradient solvent system was used.

**[Table 1]**

| | |
|---|---|
| Device name | Agilent, 1200 HPLC Chemstation |
| Column | Altima HP C18 HL (3.0×150 mm) |
| Detector | Alltech, Evaporative Light Scattering Detector (ELSD) |
| Sample amount | 20 µL |
| Solvent | Acetonitrile: Dichloromethane |
| | using gradient solvent system |
| Detector gain | 1 |
| Oven temperature of detector | 45°C |
| Carrying gas | N₂(1.5 L/min) |

### 3.2. Analysis of triglyceride composition

Analysis of triglyceride of the lipid composition, obtained through the distillation process according to Example 2, was performed with regard to its composition and content. The composition of triglyceride, identified by the HPLC-ELSD, is shown in Table 2 below.

**[Table 2]**

| Substrate ratio (PMF:stearic acid ethyl ester) | Reaction time | POS Content (%) | SUS Content (%) | POS/SUS |
|---|---|---|---|---|
| 1:5 | 3H | 30.3 | 79.5 | 0.38 |
| 1:4 | 3H | 36.7 | 77.3 | 0.47 |
| 1:4 | 5H | 45.9 | 75.2 | 0.61 |
| 1:3 | 3H | 38.6 | 74.9 | 0.52 |
| 1:2 | 5H | 43.2 | 74.4 | 0.58 |
| 1:2 | 6H | 47.5 | 73.2 | 0.65 |
| 1:1.5 | 10H | 50.2 | 70.3 | 0.71 |
| 1:1 | 12H | 51.8 | 70.0 | 0.74 |

As shown in Table 2 above, it was confirmed that lipid compositions having the POS content of at least 30% and a weight percent ratio of POS to SUS (POS/SUS) of 0.38 to 0.74 were prepared, depending on reaction substrate ratios and time.

### Example 4: SFC analysis of lipid composition using nuclear magnetic resonance (NMR)

### 4.1. NMR analysis

NMR analysis conditions for analyzing the SFC of the lipid composition were based on ISO 8292-2, and specific conditions are shown in Table 3 below. An SFC analysis test using NMR was conducted by a parallel method. Specifically, five 3 mL samples were prepared, and the lipids were sufficiently melted at 80°C in pre-treatment, followed by cooling at 60°C for 10 minutes and then at 0°C for 90 minutes. Then, the crystals were stabilized at 26°C for 40 hours, and then cooled at 0°C for 90 minutes. The samples were left for 30 minutes in a Celsius bath with a metal block thermostat preset to 10.0°C, 20.0°C, 25.0°C, 30.0°C, and 35.0°C, respectively, and then used for the NMR analysis. The measuring time was about 6 seconds.

**[Table 3]**

| | |
|---|---|
| NMR Device Names | Minispec, BRUKER |
| Frequency | 60 MHz |
| Sample amount | 3 µL |
| Pre-treatment temperature | 100 metal block thermostat, 0°C |
| Experiment temperatures | 10.0°C, 20.0°C, 25.0°C, 30.0°C, 35.0°C |

### 4.2. Solid fat content analysis

Solid fat content (SFC) of the four types of the lipid compositions prepared according to Example 2, and shea butter and sunflower oil as comparative examples were analyzed. The four lipid compositions were labeled as A to D. Specifically, lipid composition A has an SUS content of 79.5% and a POS/SUS ratio of 0.38; B has an SUS content of 75.2% and a POS/SUS ratio of 0.61; C has an SUS content of 74.4% and a POS/SUS ratio of 0.58; and D an SUS content of 70.0% and a POS/SUS ratio of 0.74.

Shea butter used as a comparative example is a material widely used as a cosmetic raw lipid and contains 45 wt% of SUS and has a POS/SUS ratio of 0.1.

The SFCs measured on the four types of the lipid compositions, shea butter, and sunflower oil are shown in Table 4 below.

**[Table 4]**

| | Solid fat content (%) | | | |
|---|---|---|---|---|
| | 20°C | 25°C | 30°C | 35°C |
| Lipid composition A | 67.1 | 58.4 | 36.7 | 3.2 |
| Lipid composition B | 73.0 | 63.4 | 45.3 | 3.3 |
| Lipid composition C | 65.1 | 60.1 | 40.1 | 2.8 |
| Lipid composition D | 62.8 | 57.6 | 38.9 | 2.5 |
| Shea butter | 44.3 | 40.6 | 33.2 | 11.3 |
| Sunflower oil | 0 | 0 | 0 | 0 |

As shown in Table 4 above, except the sunflower oil containing no SFC in the entire temperature range, lipid compositions A to D and shea butter tend to have decreasing SFC as the temperature increases. In particular, the SFC of lipid compositions A to D of the present disclosure drastically decreased within the temperature range of 30°C to 35°C, which is close to a skin temperature, compared to shea butter, the comparative example. Specifically, the SFC of shea butter decreased from 33.2% to 11.3% as the temperature increased from 30°C to 35°C, whereas the SFC of the lipid compositions of the present disclosure markedly decreased to around 3% at 35°C.

Additionally, as the lipid compositions of the present disclosure have a high SFC at room temperature (20°C to 25°C), they can maintain a stable state despite long-term delivery and storage. In contrast, lipids such as shea butter having a low SFC are highly likely to be oxidized according to packing conditions, and therefore have low oxidation stability.

### Example 5: Property diversification of lipid composition

### 5.1. Preparation of lipid composition

Lipid compositions containing various SFCs were prepared by mixing palm olein, which is a vegetable lipid having a low SFC, into the lipid composition obtained through the distillation according to Example 2. Specifically, lipid compositions E to G were prepared using lipid compositions C and D used in Example 4 and palm olein at different composition ratios. The composition ratios, SUS contents, and POS/SUS weight ratios of lipid compositions E to G are shown in Table 5 below. Lipid compositions C and D are according to the present invention.

**[Table 5]**

| | Lipid composition C (wt%) | Lipid composition D (wt%) | Palm olein (wt%) | SUS content (wt%) | POS/SUS |
|---|---|---|---|---|---|
| Lipid composition C | 100 | - | 0 | 74.4 | 0.58 |
| Lipid composition D | - | 100 | 0 | 70 | 0.74 |
| Lipid composition E | 90 | - | 10 | 71.2 | 0.58 |
| Lipid composition F | 80 | - | 20 | 63.2 | 0.58 |
| Lipid composition G | 70 | - | 30 | 55.3 | 0.58 |
| Lipid composition H | - | 70 | 30 | 49 | 0.74 |
| Lipid composition I | - | 60 | 40 | 42 | 0.74 |
| Shea butter | - | - | - | 45 | 0.1 |
| Sunflower oil | - | - | - | - | - |

### 5.2. SFC analysis of lipid composition

Solid fat contents of lipid compositions E to I prepared according to Examples 5.1, shea butter, and sunflower oil were analyzed. The SFC analysis was conducted using the same method as Example 4.1, and the result is shown in Table 6 below.

**[Table 6]**

| | Solid fat content (%) | | | |
|---|---|---|---|---|
| | 20°C | 25°C | 30°C | 35°C |
| Lipid composition C | 65.1 | 60.1 | 40.1 | 2.8 |
| Lipid composition D | 62.8 | 57.6 | 38.9 | 2.5 |
| Lipid composition E | 66.3 | 58.9 | 37.4 | 2.8 |
| Lipid composition F | 58.9 | 50.1 | 26.9 | 2.5 |
| Lipid composition G | 50.4 | 42.6 | 21.5 | 2.1 |
| Lipid composition H | 49.1 | 37.1 | 17.8 | 1.9 |
| Lipid composition I | 45.2 | 31.2 | 15.2 | 1.5 |
| Shea butter | 44.3 | 40.6 | 33.2 | 11.3 |
| Sunflower oil | 0 | 0 | 0 | 0 |

As shown in Table 6 above, the SFC of lipid compositions C to I is 3% or less at 35°C, whereas the SFC of shea butter is about 11% at 35°C. Additionally, shea butter has a ratio of SFC at 30°C to SFC at 35°C of 2.93, whereas the lipid compositions have a ratio of SFC at 30°C to SFC at 35°C of 9 to 15. Further, in comparison of SFCs at room temperature (20°C to 25°C), the SFC of the lipid compositions is higher than the SFC of shea butter. In other words, as the lipid compositions have a high SFC at room temperature, the solid formulation thereof can remain stable. The lipid compositions melt at 35°C, which is close to a skin temperature, and thus have no uncomfortable sensation due to residues upon application to the skin. In contrast, the SFC of shea butter is much higher than that of the lipid compositions at 35°C, and is low at room temperature. Therefore, compared to shea butter, the lipid compositions have better delivery and shelf stability at room temperature and rapidly melt upon application to the skin, and thus are suitable for use in cosmetic compositions.

### Example 6: Stability analysis of lipid composition

To use a lipid composition for a cosmetic ingredient, the composition must maintain its color and formulation even when stored for a long period of time. In other words, such composition must have high oxidation stability, which can be evaluated by measuring a change in color values according to time. More specifically, the color value analysis can be conducted by measuring color values according to time at a constant temperature.

### 6.1. Color value analysis

An analytical experiment for the color value analysis of the lipid compositions were conducted in accordance with AOCS method Cc13b-45, ISO 15305, and specific conditions thereof are shown in Table 7 below. More specifically, about 100 mL of each sample was prepared, and the lipids were sufficiently melted at 80°C before the experiment. The samples were put in 5.25 inch cells to measure a color value thereof according to time using a color value device.

**[Table 7]**

| Color value device | Lovibond PFX 950 Tintometer |
|---|---|
| Light source | Tungsten halogen lamp (white light) |
| Sample amount | 100 mL |
| Pre-treatment temperature | 80°C |
| Experiment temperature | Room temperature (25°C) |
| Measured cell | 5.25 inch optical glass cell |

After measuring the color values as shown in Table 7 above, the values can be analyzed with respect to red (R) and yellow (Y) values in accordance with the AOCS or Lovibond method.

### 6.2. Evaluation of stability associated with storage of lipid compositions

In order to evaluate the stability of lipid compositions C and G prepared according to Examples 2 and 5 and shea butter, lipid compositions C and G, and shea butter were allowed to stand at 25°C for five weeks, and then color values thereof were measured each week to be analyzed.

The color values were analyzed according to Example 6.1 using the ACOS color method, and were compared with respect to the R value. Lipid composition C is according to the present invention.

**[Table 8]**

| | Week 0 | Week 1 | Week 2 | Week 3 | Week 4 | Week 5 |
|---|---|---|---|---|---|---|
| Lipid composition C | 1.4 R | 1.4 R | 1.4 R | 1.4 R | 1.5 R | 1.5 R |
| Lipid composition G | 1.5 R | 1.5 R | 1.5 R | 1.6 R | 1.6 R | 1.6 R |
| Shea butter | 1.7 R | 1.8 R | 1.9 R | 1.9 R | 2.0 R | 2.1 R |
| As shown in Table 8 above, the lipid compositions C and G of the present disclosure rarely changed their colors even though they were allowed to stand for a long period of time, | | | | | | |

whereas shea butter exhibited a relatively large change in its color values. In other words, the lipid compositions of the present disclosure were confirmed to have high stability at room temperature compared to shea butter.

### 6.3. Stability evaluation of lipid compositions at high temperature

In order to evaluate the stability of lipid compositions C and G prepared according to Examples 2 and 5, and shea butter at a high temperature, they were heated at 80°C for six hours, and then color values thereof were measured to be analyzed.

The color values were analyzed according to Example 6.1 using the ACOS color method, and were compared with respect to the R value. Lipid composition C is according to the present invention.

**[Table 9]**

| | 0 hours | 6 hours |
|---|---|---|
| Lipid composition C | 1.4 R | 1.6 R |
| Lipid composition G | 1.5 R | 1.8 R |
| Shea butter | 1.7 R | 2.3 R |

As shown in Table 9 above, lipid compositions C and G did not exhibit a significant change in their color values after being exposed to 80°C for six hours, compared to shea butter. In other words, the lipid compositions of the present disclosure were confirmed to have high oxidation stability even if they were exposed to the air at a high temperature, compared to shea butter.

### Example 7: Evaluation of cosmetic application: lip balm

### 7.1. Preparation of lip balm

The lipid compositions of the present disclosure were applied to conventional lip balm mixtures and then formulated. Lipid compositions C, F, and I, which exhibited a wide variation of the SFC depending on temperatures, were used as experimental groups, and shea butter and sunflower oil, which are used for butter and a liquid oil, respectively, in existing cosmetic products were used as comparative groups to prepare lip balm. Lip balm was prepared with the same compositions for both groups. Specific raw materials and composition ratio used for the lip balm preparation are shown in Table 10 below. Lipid composition C is according to the present invention.

**[Table 10]**

| Raw material | Lip balm C | Lip balm F | Lip balm I | Lip balm -shea butter | Lip balm - sunflower oil |
|---|---|---|---|---|---|
| Lipid composition C | 30 | - | - | - | - |
| Lipid composition F | - | 30 | - | - | - |
| Lipid composition I | - | - | 30 | - | - |
| Shea butter | - | - | - | 30 | - |
| Sunflower oil | to 100 | to 100 | to 100 | to 100 | to 100 |
| Jojoba ester | 13 | 13 | 13 | 13 | 13 |
| Carnauba wax | 5 | 5 | 5 | 5 | 5 |
| Candelilla wax | 5 | 5 | 5 | 5 | 5 |
| Hydroxystearic/linoleni c/oleic polyglycerides | 3.5 | 3.5 | 3.5 | 3.5 | 3.5 |
| Tocopheryl acetate | 0.5 | 0.5 | 0.5 | 0.5 | 0.5 |
| Preservative, aromatic | Proper amount | Proper amount | Proper amount | Proper amount | Proper amount |

All raw materials were heated at 75°C to be sufficiently melted, filled into an about 5 mL cylinder at 50°C, and cooled to room temperature. The lipid compositions of the present disclosure, as shea butter and sunflower oil used in existing cosmetics, were well mixed with the other lip balm mixtures. This shows that the lipid compositions of the present disclosure have excellent miscibility with the existing cosmetic raw materials, and accordingly can be used in place of the conventional lipids such as shea butter and sunflower oil.

### 7.2. Evaluation of shelf stability of lip balm

The lip balm prepared according to Example 7.1 was stored at a specific temperature for a certain period of time and evaluated on its stability. The storage temperatures were set to 25°C and 45°C. The lip balm was observed with the naked eyes at an interval of 10 days and classified into the following three groups: ∘ for a formulation maintained without any change, △ for a formulation with partial changes such as discoloration, fragrance change, and phase separation, and × for a formation with a drastic change. The observation was continued for 30 days, and the result is shown in Table 11 below. Lipid balm C is according to the present invention.

**[Table 11]**

| | | Lip balm C | Lip balm F | Lip balm I | Lip balm -shea butter | Lip balm - sunflower oil |
|---|---|---|---|---|---|---|
| Storage at 25°C | After 10 days | ○ | ○ | ○ | ○ | ○ |
| | After 20 days | ○ | ○ | ○ | ○ | × |
| | After 30 days | ○ | ○ | ○ | ○ | × |
| Storage at 45°C | After 10 days | ○ | ○ | ○ | ○ | × |
| | After 20 days | ○ | ○ | ○ | ○ | × |
| | After 30 days | ○ | △ | △ | △ | × |

As shown in Table 11 above, the lip balm prepared to contain the lipid compositions of the present disclosure was confirmed to have stability equivalent or superior to that prepared using shea butter. The lip balm prepared using sunflower liquid oil drastically changed as the temperature and/or number of days of storage increase. This shows that the lipid compositions of the present disclosure can be used in place of lipids such as expensive shea butter, *etc.* for cosmetics such as lip balm.

### 7.3. Evaluation of applicability of lip balm to skin

The lip balm prepared according to Example 7.1 was applied directly to the skin to identify its characteristics. A panel composed of 20 men and women aged from 20 to 40 participated in the experiment. Evaluation criteria were based on 1(very dissatisfied) to 10 (very satisfied), and the sensory test results according to each evaluation item are summarized in Table 12 below. Lipid balm C is according to the present invention.

**[Table 12]**

| | Lip balm C | Lip balm F | Lip balm I | Lip balm -shea butter | Lip balm - sunflower oil |
|---|---|---|---|---|---|
| Ease in the skin application | 8.5 | 8.0 | 8.2 | 8.0 | 6.5 |
| Adherent sensation | 9.2 | 8.4 | 7.6 | 8.2 | 5.1 |
| Moist sensation | 8.6 | 8.0 | 8.6 | 8.1 | 6.8 |
| Sensation for melting speed at a skin temperature | 8.5 | 8.3 | 8.1 | 6.8 | 7.1 |

As shown in Table 12 above, the lip balm prepared to contain the lipid compositions of the present disclosure was confirmed to have ease in application to skin, equivalent or superior to that prepared using shea butter. Additionally, the lip balm prepared to contain the lipid compositions of the present disclosure exhibited adherent and moist sensations superior to that prepared using shea butter, and had excellent melting speed by a skin temperature. In contrast, the lip balm prepared using sunflower liquid oil overall showed inferior ease in the skin application, skin adhesion, and moist sensations.

### Example 8: Applicability evaluation as cosmetic formulation: cream

### 8.1. Preparation of cream

The lipid compositions of the present disclosure were applied to conventional cream mixtures and then formulated. Lipid compositions C, F, and I, which exhibited a wide variation in the SFC depending on temperatures, were used as experimental groups, and shea butter and sunflower oil, which are used for butter and a liquid oil, respectively, in existing cosmetics, were used as comparative groups to prepare cream. Specific raw materials and composition ratio used for the cream preparation are shown in Table 13 below.

**[Table 13]**

| Raw material | Cream C | Cream F | Cream I | Cream -shea butter | Cream - sunflower oil |
|---|---|---|---|---|---|
| Purified water | to 100 | to 100 | to 100 | to 100 | to 100 |
| Carbomer | 0.2 | 0.2 | 0.2 | 0.2 | 0.2 |
| Xanthan gum | 0.1 | 0.1 | 0.1 | 0.1 | 0.1 |
| Butylene glycol | 5 | 5 | 5 | 5 | 5 |
| Glycerin | 3 | 3 | 3 | 3 | 3 |
| Lipid composition C | 20 | - | - | - | - |
| Lipid composition F | - | 20 | - | - | - |
| Lipid composition I | - | - | 20 | - | - |
| Shea butter | - | - | - | 20 | - |
| Sunflower oil | - | - | - | - | 20 |
| Caprylic and capric triglyceride | 5 | 5 | 5 | 5 | 5 |
| Glyceryl stearate PEG-100 stearate | 2 | 2 | 2 | 2 | 2 |
| Cetearyl alcohol | 1.5 | 1.5 | 3.5 | 1.5 | 1.5 |
| Ceteareth-12 | 0.5 | 0.5 | 0.5 | 0.5 | 0.5 |
| Arginine | 0.2 | 0.2 | 0.2 | 0.2 | 0.2 |
| Preservative, aromatic | Proper amount | Proper amount | Proper amount | Proper amount | Proper amount |

All raw materials above were heated to 75°C to be sufficiently melted, filled into an about 50 mL glass bottle at 40°C to 45°C, and cooled to room temperature. The lipid compositions of the present disclosure, as shea butter and sunflower oil used for conventional cosmetics, were mixed well with the lip balm mixture. This shows that the lipid compositions of the present disclosure have excellent miscibility with the existing cosmetics, and accordingly can be used in place of the conventionally used lipids, such as shea butter and sunflower oil.

### 8.2. Evaluation of shelf stability of cream

The cream prepared according to Example 8.1 was stored at a specific temperature for a certain period of time and evaluated on its stability. The storage temperatures were set to 25°C and 45°C. The cream was observed with the naked eye at an interval of 20 days, and classified into the following three groups: ∘ for a formulation maintained without any change, △ for a formulation with partial changes such as discoloration, fragrance change, and phase separation, and × for a formation with a drastic change. The observation continued for 60 days, and the result is shown in Table 14 below. Cream C is according to the present invention.

**[Table 14]**

| | | Cream C | Cream F | Cream I | Cream -shea butter | Cream - sunflower oil |
|---|---|---|---|---|---|---|
| Storage at 25°C | After 20 days | ○ | ○ | ○ | ○ | ○ |
| | After 40 days | ○ | ○ | ○ | ○ | ○ |
| | After 60 days | ○ | ○ | ○ | ○ | △ |
| Storage at 45°C | After 20 days | ○ | ○ | ○ | ○ | △ |
| | After 40 days | ○ | ○ | △ | △ | △ |
| | After 60 days | ○ | △ | △ | △ | × |

As shown in Table 14 above, the cream prepared containing the lipid compositions of the present disclosure was confirmed as having stability equivalent or superior to that prepared using shea butter, whereas the cream prepared using sunflower liquid oil exhibited insufficient shelf stability. This shows that the lipid compositions of the present disclosure can be used in place of lipids such as expensive shea butter, *etc.* for cosmetics such as cream.

### 8.3. Evaluation of skin applicability of cream

The cream prepared according to Example 8.1 was applied directly to the skin to identify its characteristics. A panel composed of 20 men and women aged from 20 to 40 participated in the experiment. Evaluation criteria were based on 1(very dissatisfied) to 10 (very satisfied), and the sensory test results according to each evaluation item are summarized in Table 15 below. Cream C is according to the present invention.

**[Table 15]**

| | Cream C | Cream F | Cream I | Cream -shea butter | Cream - sunflower oil |
|---|---|---|---|---|---|
| Ease in the skin application | 8.3 | 8.0 | 8.2 | 7.3 | 6.8 |
| Adherent sensation | 9.2 | 8.4 | 7.6 | 8.2 | 5.1 |
| Moist sensation | 8.6 | 8.0 | 8.6 | 8.1 | 6.8 |
| Stickiness | 8.8 | 8.6 | 8.3 | 7.0 | 6.5 |
| Sensation for melting speed at a skin temperature | 8.5 | 8.3 | 8.1 | 6.8 | 7.1 |

As shown in Table 15 above, the cream prepared to contain the lipid compositions of the present disclosure was confirmed to have excellent ease in skin application as well as equivalent or superior skin adhesion and moisture compared to that prepared using shea butter. Further, the cream prepared by including the lipid compositions of the present disclosure was superior to the others in terms of stickiness and rapid melting feeling at the skin temperature. In other words, the cream prepared containing the lipid compositions of the present disclosure is rapidly melted in response to the skin temperature upon its application to the skin, having generally excellent sensations, such as little uncomfortable sensation due to residues, excellent touching sensation, and little stickiness along with rapid absorption to skin.

## Claims

1. A lipid composition comprising at least 40 wt% of saturated acid-unsaturated acid-saturated acid (SUS) based on the total weight of the lipid composition, wherein the SUS comprises 58 parts by weight to 74 parts by weight of 1-palmitoyl-2-oleoyl-3-stearoyl glycerol (POS) based on 100 parts by weight of SUS,
wherein the lipid composition further comprises a vegetable lipid which comprises 0 wt% to 5 wt% of solid fat content (SFC) at 30°C, and
wherein a ratio of solid fat content (SFC) at a temperature of 20°C to 25°C compared to that of SFC at 35°C is 10 to 35.

2. The lipid composition of claim 1, wherein the vegetable lipid is at least one selected from the group consisting of palm oil, palm olein, palm stearin, shea olein, and shea stearin.

3. A cosmetic composition comprising the lipid composition of claim 1.

4. A method for preparing the lipid composition of claim 1, comprising performing transesterification by mixing a fatty acid or a derivative thereof; a vegetable lipid; and lipase.

5. The method of claim 4, wherein the fatty acid or a derivative thereof is at least one selected from the group consisting of stearic acid, stearic acid ethyl ester, and stearic acid methyl ester.

6. The method of claim 4, wherein the vegetable lipid is at least one selected from the group consisting of palm kernel oil, palm oil, and sunflower oil.

7. The method of claim 4, wherein the lipase is derived from Mucor miehei.

8. The method of claim 4, further comprising, after the transesterification, adding a vegetable lipid which comprises 0 wt% to 5 wt% of SFC at 30°C.

## Patentansprüche

1. Lipidzusammensetzung, umfassend mindestens 40 Gew.-% Gesättigte-Säure/Ungesättigte-Säure/Gesättigte-Säure (SUS), bezogen auf das Gesamtgewicht der Lipidzusammensetzung, wobei die SUS 58 Gewichtsteile bis 74 Gewichtsteile 1-Palmitoyl-2-oleoyl-3-stearoylglycerin (POS) umfasst, bezogen auf 100 Gewichtsteile SUS,
wobei die Lipidzusammensetzung ferner ein pflanzliches Lipid umfasst, das bei 30°C 0 Gew.-% bis 5 Gew.-% Festfettgehalt (SFC) umfasst, und
wobei ein Verhältnis von Festfettgehalt (SFC) bei einer Temperatur von 20°C bis 25°C verglichen mit jenem eines SFC bei 35°C 10 bis 35 beträgt.

2. Lipidzusammensetzung nach Anspruch 1, wobei das pflanzliche Lipid mindestens eines ist, das aus der Gruppe bestehend aus Palmöl, Palmolein, Palmstearin, Sheaolein und Sheastearin ausgewählt ist.

3. Kosmetische Zusammensetzung, umfassend die Lipidzusammensetzung nach Anspruch 1.

4. Verfahren zur Herstellung der Lipidzusammensetzung nach Anspruch 1, umfassend ein Durchführen einer Umesterung durch Mischen von einer Fettsäure oder einem Derivat davon; einem pflanzlichen Lipid; und Lipase.

5. Verfahren nach Anspruch 4, wobei die Fettsäure oder ein Derivat davon mindestens eine bzw. eines ist, die bzw. das aus der Gruppe bestehend aus Stearinsäure, Stearinsäureethylester und Stearinsäuremethylester ausgewählt ist.

6. Verfahren nach Anspruch 4, wobei das pflanzliche Lipid mindestens eines ist, das aus der Gruppe bestehend aus Palmkernöl, Palmöl und Sonnenblumenöl ausgewählt ist.

7. Verfahren nach Anspruch 4, wobei die Lipase von Mucor miehei abgeleitet ist.

8. Verfahren nach Anspruch 4, ferner umfassend nach der Umesterung ein Zugeben eines pflanzlichen Lipids, das bei 30°C 0 Gew.-% bis 5 Gew.-% SFC umfasst.

## Revendications

1. Composition lipidique comprenant au moins 40 % en poids d'acide saturé-acide insaturé-acide saturé (SUS) par rapport au poids total de la composition lipidique, dans laquelle le SUS comprend de 58 parties en poids à 74 parties en poids de 1-palmitoyl-2-oléoyl-3-stéaroyl glycérol (POS) pour 100 parties en poids de SUS,
dans laquelle la composition lipidique comprend en outre un lipide végétal qui présente une teneur de 0 % à 5 % en poids en graisse solide (SFC) à 30 °C, et
dans laquelle le rapport entre la teneur en graisse solide (SFC) à une température de 20 °C à 25 °C à celle à 35 °C va de 10 à 35.

2. Composition lipidique selon la revendication 1, dans laquelle le lipide végétal est au moins un élément choisi parmi le groupe constitué de l'huile de palme, de l'oléine de palme, de la stéarine de palme, de l'oléine de karité et de la stéarine de karité.

3. Composition cosmétique comprenant la composition lipidique selon la revendication 1.

4. Procédé de préparation de la composition lipidique selon la revendication 1, comprenant la réalisation d'une transestérification par mélange d'un acide gras ou d'un dérivé de celui-ci, d'un lipide végétal et d'une lipase.

5. Procédé selon la revendication 4, dans lequel l'acide gras ou un dérivé de celui-ci est au moins un élément choisi parmi le groupe constitué de l'acide stéarique, de l'ester éthylique d'acide stéarique et de l'ester méthylique d'acide stéarique.

6. Procédé selon la revendication 4, dans lequel le lipide végétal est au moins un élément choisi dans le groupe constitué par l'huile de palmiste, l'huile de palme et l'huile de tournesol.

7. Procédé selon la revendication 4, dans lequel la lipase est dérivée de Mucor miehei.

8. Procédé selon la revendication 4, comprenant en outre, après la transestérification, l'ajout d'un lipide végétal comprenant de 0 % en poids à 5 % en poids de SFC à 30 °C.
